# EUROPEAN PATENT APPLICATION

(11) **EP 4 848 308 A1**
(43) Date of publication of application: **02.12.2026**
(21) Application number: 26181891.8
(22) Date of filing: 29.05.2026
(51) Int. Cl.: G16H 50/30, A61B 5/11, A61B 5/024, G16H 30/40, G16H 50/20

(54) **ARTIFICIAL INTELLIGENCE HEALTH MONITORING APPLICATION**

(30) Priority: 29.05.2025 US 202563814155 P; 25.03.2026 US 202619578645
(71) Applicant: Caremindr Corporation, Los Gatos, CA 95032 (US)
(72) Inventor: SOZA, Harry Raymond, San Jose, 95126 (US); YERMILOV, Irina, Los Angeles, 90045 (US); COATNEY, Randall Edwin, Lafayette, 80026 (US)
(74) Representative: Cobbold, Alistair John

(57) **Abstract**

An example operation includes receiving one or more user face images of a user from a user health application, determining a user cardiovascular score based on the one or more user face images, retrieving a previously stored user health score, transmitting one or more questions to the user health application to solicit current user health information, determining a current user health score based on one or more answers to the one or more questions, and determining a current user health status based on the user cardiovascular score, the previously stored user health score and the current user health score.

## Description

### FIELD OF INVENTION

Various embodiments relate generally to using artificial intelligence (AI) to monitor and provide health assistance via a user application.

### BACKGROUND

Conventionally, patients may attend a medical appointment and receive medical care and/or advice. The patient is generally not monitored or contacted once the appointment has ended and any necessary follow-up sessions have commenced. A user application operated on a user device may provide added structure to a personal health care gameplan and provide monitoring functions which can be used to identify whether a person needs any immediate attention or related services.

### SUMMARY

The appended claims may serve as a summary of one or more embodiments of a machine learning platform ore related artificial intelligence application.

Further areas of applicability of the present disclosure will become apparent from the detailed description, the claims and the drawings. The detailed description and specific examples are intended for illustration only and are not intended to limit the scope of the disclosure.

An example operation includes receiving one or more user face images of a user from a user health application, determining a user cardiovascular score based on the one or more user face images, retrieving a previously stored user health score, transmitting one or more questions to the user health application to solicit current user health information, determining a current user health score based on one or more answers to the one or more questions, and determining a current user health status based on the user cardiovascular score, the previously stored user health score and the current user health score.

### BRIEF DESCRIPTION OF THE DRAWINGS

The present invention relates generally to a health monitoring application which uses current cardiovascular data measured by video and image analysis and considers other data such as user answer data, previous health related data, etc., and determines whether immediate health care or related care is necessary to alleviate the health care needs of the patient/user.

The present disclosure will become better understood from the detailed description and the drawings, wherein:
FIG. 1A illustrates an example user interface used for recording content related to a user's face according to example embodiments;
FIG. 1B illustrates an example user interface used to examine various digital frames of a user's face for cardiovascular data according to example embodiments;
FIG. 1C illustrates an example user interface of a process of generating a cardiovascular data report according to example embodiments;
FIG. 1D illustrates an example user interface of the cardiovascular data report results according to example embodiments;
FIG. 1E illustrates an example user interface of a health data question template according to example embodiments;
FIG. 2 illustrates an example of a data collecting and processing model used for creating a data report and determining whether a user is having medical problems according to example embodiments;
FIG. 3 illustrates an example of a data collecting and processing procedure used for collecting data and outputting a medical report based on a machine learning model(s) according to example embodiments;
FIG. 4 illustrates an example logic diagram illustrating information and data processing modules used to measure and monitor according to example embodiments;
FIG. 5 illustrates an example flow diagram of an example process of measuring and monitoring patient health according to example embodiments; and
FIG. 6 illustrates an example computing platform used to perform one or more operations associated with example embodiments.

### DETAILED DESCRIPTION

It will be readily understood that the instant components, as generally described and illustrated in the figures herein, may be arranged and designed in a wide variety of different configurations. Thus, the following detailed description of the embodiments of at least one of a method, apparatus, computer readable storage medium and system, as represented in the attached figures, is not intended to limit the scope of the application as claimed but is merely representative of selected embodiments. Multiple embodiments depicted herein are not intended to limit the scope of the solution. The computer-readable storage medium may be a non-transitory computer readable media or a non-transitory computer readable storage medium.

The instant features, structures, or characteristics described in this specification may be combined in any suitable manner in one or more embodiments. For example, the usage of the phrases "example embodiments," "some embodiments," or other similar language, throughout this specification refers to the fact that a particular feature, structure, or characteristic described in connection with the embodiment may be included in at least one example. Thus, appearances of the phrases "example embodiments", "in some embodiments", "in other embodiments," or other similar language, throughout this specification can all refer to the same embodiment. Thus, these embodiments may work in conjunction with any of the other embodiments, may not be functionally separate, and the described features, structures, or characteristics may be combined in any suitable manner in one or more embodiments.

Example embodiments provide methods, systems, hardware components, non-transitory computer readable media, devices, and/or networks, which provide for a monitoring platform that identifies user health concerns and returns results which include current health status based on the input data and retrieved data received and processed from various information sources.

A contactless, artificial intelligence (AI) health monitoring application operates by analyzing real-time facial images/video data to extract a wide range of biometric and wellness indicators. Multimodal sensing may be used, including but not limited to photoplethysmography (PPG), ballistocardiography (BCG), and vascular flow analysis. The resulting data may be integrated with known user health data and data extracted from question and answer sessions with the user to provide an instant result.

In operation, the multimodal sensing portion may generate high-fidelity health metrics, such as heart rate, heart rate variability, stress index, blood pressure, and cardiovascular risk scores. The metrics may be computed on the user's mobile device and/or in a secure server in the cloud and shared with the user device to preserve privacy. The combination of results may be grouped into a weighted function and/or multivariable expression (function) that provides an overall score based on a combination of the data points to produce a reference value, which may be compared to a threshold value. The result of the comparison may yield a yes or no answer to the question of whether immediate medical services are needed, such as a doctor appointment, emergency room visit, call 911, etc.

The decision process as to what the user should do based on the data screening may be based on adaptive thresholds associated with personalized baselines and longitudinal trends in the medical industry. The AI engine used to estimate health scores may be trained on a diverse dataset of facial scans paired with clinical-grade ground truth, which enables robust performance across skin tones, lighting conditions, and other environments. The instant application may include both user-directed and autonomous embodiments that support dynamic triage, behavioral nudging, clinician alerts, and multi-specialist routing.

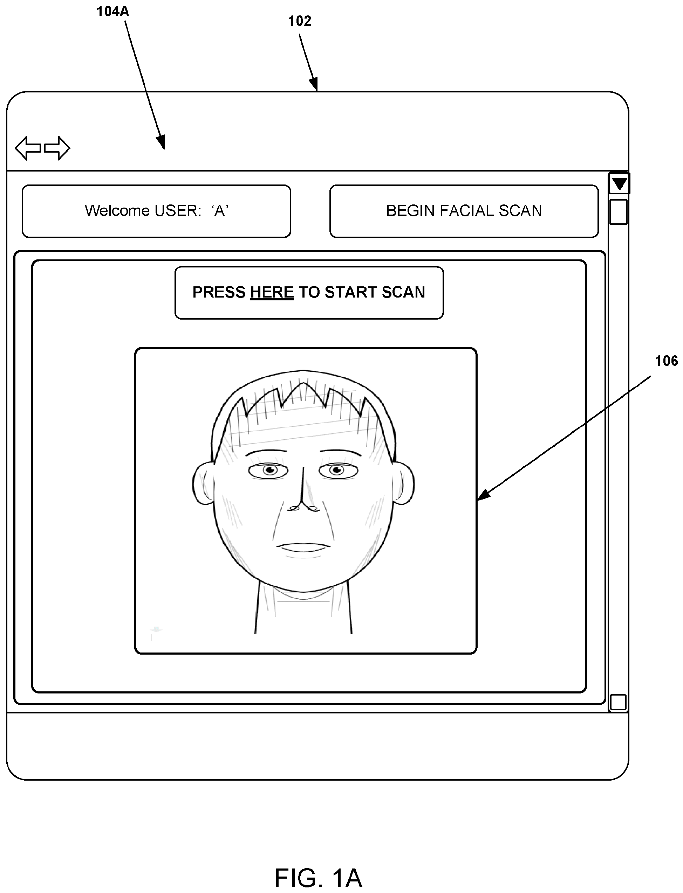

FIG. 1A illustrates an example user interface used for recording content related to a user's face according to example embodiments. Referring to FIG. 1A, the process begins with the capture of bio-scan data through a video-based facial assessment, producing physiological measurements such as blood pressure, heart rate, cardiac workload, and other health indices. In FIG. 1A, the user device 102 may have a user interface 104A which is currently operating the health application. The process may begin with a camera based user facial scan, which may be a short video (e.g., 20 seconds or less) or a series of images which are captured, stored and processed to measure cardiovascular data. A user may aim the face at the camera 106 and initiate the capture sequence. The camera may start a series of image captures, video captures, zoom in and zoom out operations, color change operations, perspective changes, etc.

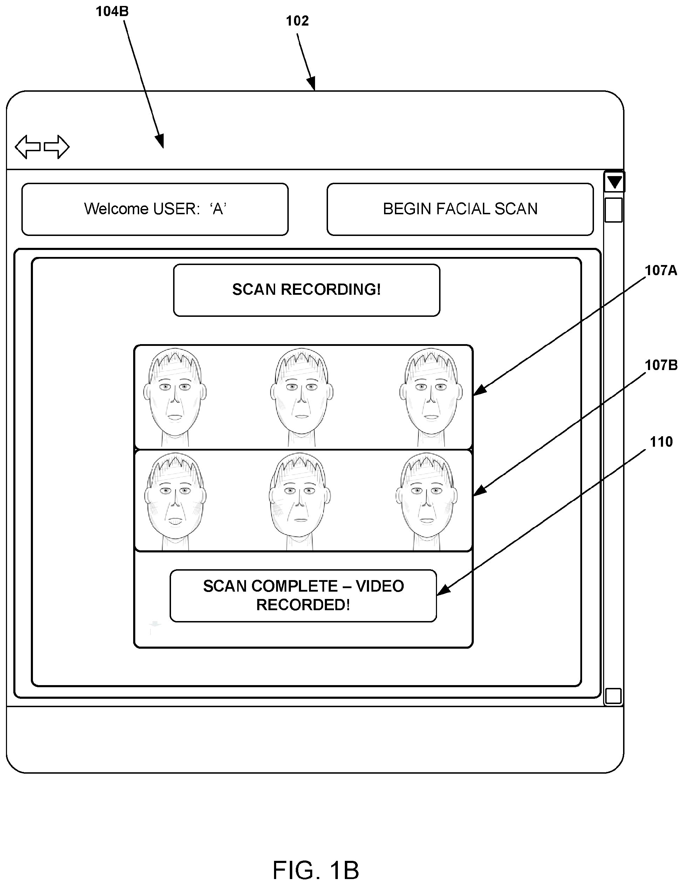

FIG. 1B illustrates an example user interface used to examine various digital frames of a user's face for cardiovascular data according to example embodiments. Referring to FIG. 1B, the user interface application example 104B illustrates the different steps, such as beginning the scanning and loading user images 107A as a first set of images based on the recording, and additional sets of images 107B based on the recording. The image data is produced from the scanning process of the user holding the user device in a position where it can capture images and video of the user's face. A prompt 110 may inform the user the scan operation is completed.

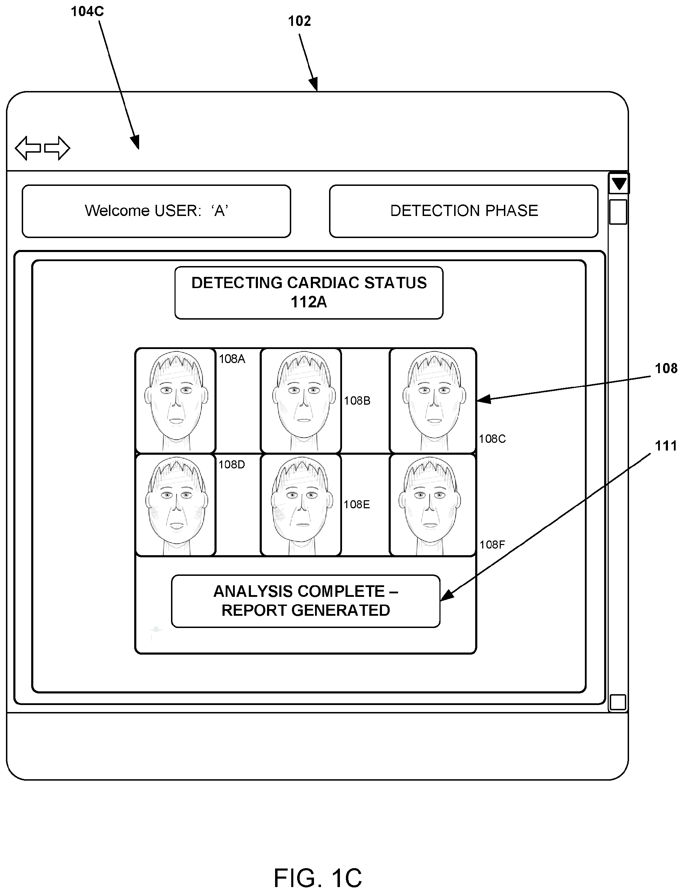

FIG. 1C illustrates an example user interface of a process of generating a cardiovascular data report according to example embodiments. Referring to FIG. 1C, the application interface 104C illustrates the images 108A-108F being extracted from a video into a set of images 108 which are analyzed for facial expressions and blood flow related data to detect a user's cardiac status 112A. A user may be moving around to some degree and the image data may be different for each frame. The image data may be analyzed for blood flow and related cardiovascular data that is used to determine data metrics. The analysis may be performed 111 by measuring changes and status information for each frame. One set of image frames may be examined at a time. Each set of images may include 'N' frames and the frames may be extracted from a captured video.

The captured video frames are streamed into a real-time multimodal signal processing pipeline that extracts distinct biosignal modalities using parallelized processing threads. The photoplethysmography (PPG) maybe computed first by measuring temporal variations in the red, green, and blue (RGB) pixel intensities associated with blood volume fluctuations in subcutaneous vessels as observed from the image data. Next, ballistocardiography (BCG) captures micro-vibrational motion vectors caused by mechanical displacement of the facial surface during cardiac ejection phases, which are analyzed using optical flow and micro-jitter tracking algorithms. Then, vascular flow mapping leverages dynamic contrast and texture gradients to estimate both the direction and velocity of capillary blood flow using localized pattern decomposition and principal component tracking.

Each modality of the process undergoes preprocessing operations via digital signal enhancement (including spatial filtering and motion compensation) followed by quality scoring. A signal quality assessment module calculates a confidence index based on metrics such as signal-to-noise ratio, frame continuity, brightness uniformity, and occlusion probability. These indices are evaluated per frame and passed to a signal fusion engine 210, which determines whether to select a dominant modality or perform weighted multimodal integration. This determination is made through a policy selector governed by a lightweight convolutional neural network trained on tens of thousands of labeled signal quality scenarios. When an option is identified, the engine applies adaptive weights to each modality's output based on real-time signal stability and environmental robustness, generating a composite biosignal time series for analysis.

In operation, the signals are provided to AI-powered analytical modules, each responsible for deriving specific health indices. Heart rate is extracted through temporal peak detection and verified by cross-spectrum analysis across modalities. Heart rate variability (HRV) is calculated using inter-beat intervals and characterized by both time-domain features (e.g., SDNN) and frequency-domain features (e.g., LF/HF power ratios). Pulse transit time (PTT), required for estimating systolic and diastolic blood pressure, is determined by analyzing pulse arrival delays across facial landmarks, which is calibrated using user demographic models and regression-based normalization derived from clinical training data. The stress index is computed using a method, such as a variant of Baevsky's histogram morphology method, incorporating quartile dispersion and distribution asymmetry. Parasympathetic activity is calculated from the high-frequency power component in the HRV spectral domain, normalized by total HRV power. Cardiac workload is estimated as the product of heart rate and systolic pressure, forming a rate-pressure product (RPP) in units of mmHg based on beats per minute. A segment selection module identifies the most stable 30-second window from the scan for each derived metric, using change point detection and noise sensitivity thresholds.

All computations may be performed locally on the user's device 102 using a compiled runtime program optimized with single instruction multiple data (SIMD) acceleration and GPU offloading where supported. The architecture is designed to ensure no biometric video data is stored or transmitted, meeting stringent data privacy standards, including GDPR and HIPAA compliance measures.

Continuing with the same example, the biometric data captured is received with structured patient-reported responses to a set of guided question and answer prompts. These inputs are compiled into a longitudinal data matrix comprising elements across multiple temporal windows (e.g., 10-day, 30-day, 90-day periods). Each individual data element, whether biometric or patient-reported, is assigned a classification tag. These tags fall into one of four categories: "OK", "Not OK", "Bad", or "Danger", representing increasing degrees of clinical concern. The tagged matrix is visualized as a color-coded grid, with each square reflecting the status of a health metric or response. The first may be green, then yellow, then orange then red for Danger. Any danger observation may yield immediate messaging and EMS services.

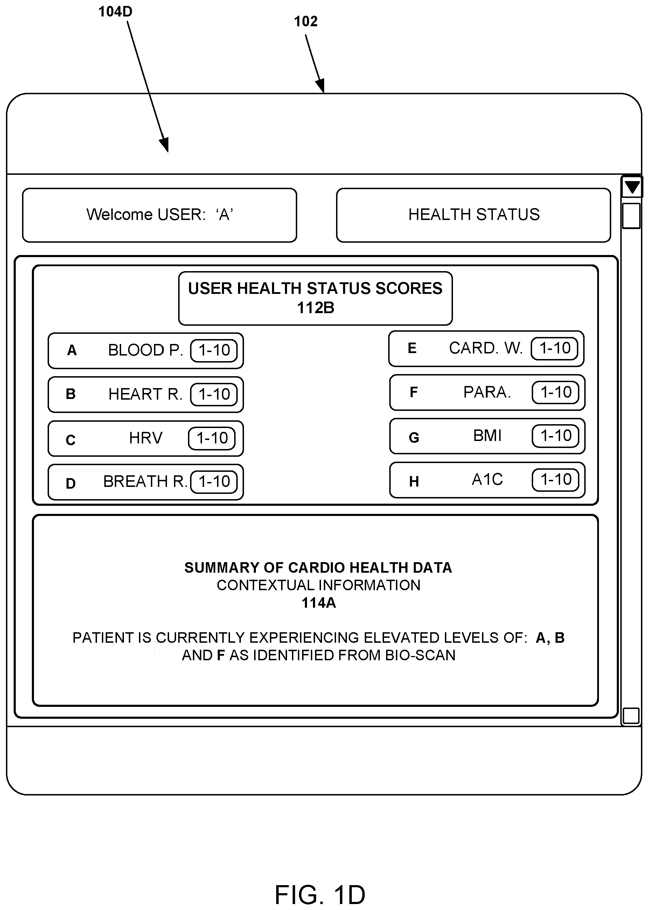

FIG. 1D illustrates an example user interface of the cardiovascular data report results according to example embodiments. Referring to FIG. 1D, the summary automatically generated for the cardiovascular screening procedure 112B may assign a value, such a 1-10 to each parameter measured or derived. The application interface 104D may include user health status scores 112B identified by A-H and including but not limited to blood pressure, heart rate, (HRV) cardiac stress, breathing rate, cardiac workload, parasympathetic activity, body mass index (BMI), and A1C levels. The summary of cardio health data 114A may identify that the patient is currently experiencing certain levels of elevated cardiovascular health based on the processed data. The values may include, in this example, values for A, B and F, the number assigned may be above 5 and as high as 10 depending on the level of concern and the numbers linked to those metrics.

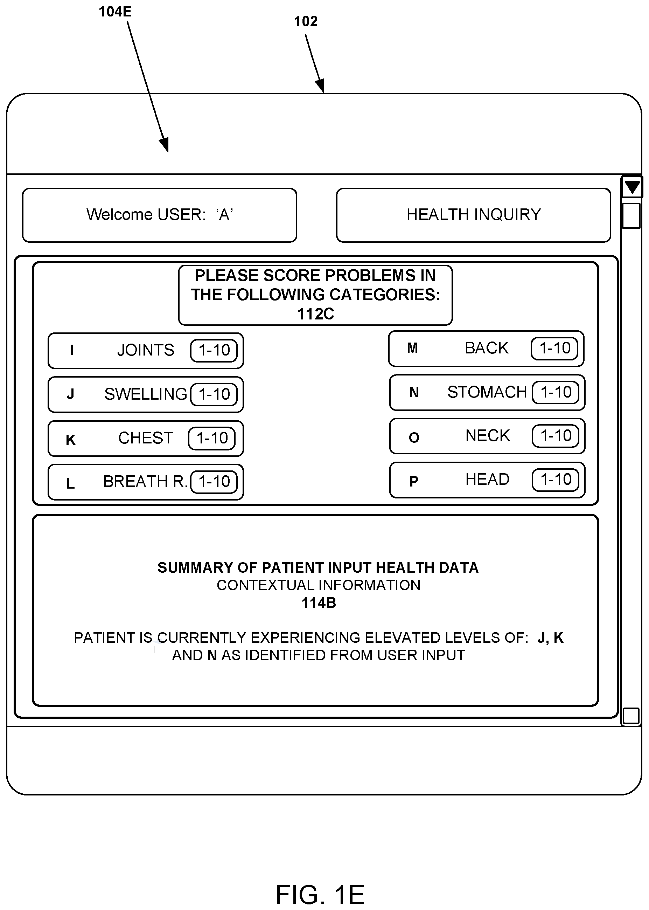

FIG. 1E illustrates an example user interface of a health data question template according to example embodiments. Referring to FIG. 1E, the application interface 104E illustrates other types of questions the application may generate for the user, such as questions outside of the cardiovascular area and related to cardiovascular health to build a broader understanding of the user's health. Example questions 112C may include questions to identify health problems include but not limited to a user's joints, swelling, chest, breathing, back, stomach, neck and head. The pains or ailments associated with one or more of the questions may provide further analysis and may provide a cross-reference analysis for the known data attributes pertaining to the user's health. The summary of patient health answers may be produced 114B to summarize the problems the user/patient is having at a particular current time.

The instant solution may select a question set based on a mapping structure stored in the memory. The mapping structure may associate a tag category and a normalized metric value with a predefined question set. The prompt generator 242 may retrieve the mapping structure and may select the question set corresponding to the consolidated tag set.

The instant solution may update the question set using a feedback vector derived from received responses. The behavioral model 244 may analyze the received responses and may generate the feedback vector, wherein the feedback vector represents a response consistency value and a response severity value. The prompt generator 242 may modify the question set based on the feedback vector and may store the modified question set in the memory for subsequent use.

A full matrix of longitudinal biometric and user answer data may be used to provide health analysis. The application may apply rule-based logic and statistical modeling to determine whether follow-on data is required. The decision may consider the severity, recency, and progression of flagged indicators. If additional clarification is needed, the application dynamically issues follow-up questions/prompts to be transmitted to the patient via the user application. Any new responses received from follow-on queries are immediately incorporated into the longitudinal matrix. These data items are re-tagged and reanalyzed by the AI processing to close the feedback loop. The cycle may iterate repeatedly until the system reaches a clinically sufficient level of certainty to provide updates and decisions to the user.

The instant solution may store the longitudinal data in a structured data matrix within the memory, wherein the structured data matrix comprises a time index, a metric index, and a value field. The processing unit 604 may write the normalized metric value and the consolidated tag set to the structured data matrix at a position defined by the time index and the metric index.

The instant solution may determine a trend value using the structured data matrix by applying a trend function to a sequence of normalized metric values associated with the time index. The processing unit 604 may retrieve the sequence from the structured data matrix and may apply the trend function to produce the trend value, wherein the trend value may be transmitted to the AI health analytics engine 220 for inclusion in the feature tensor.

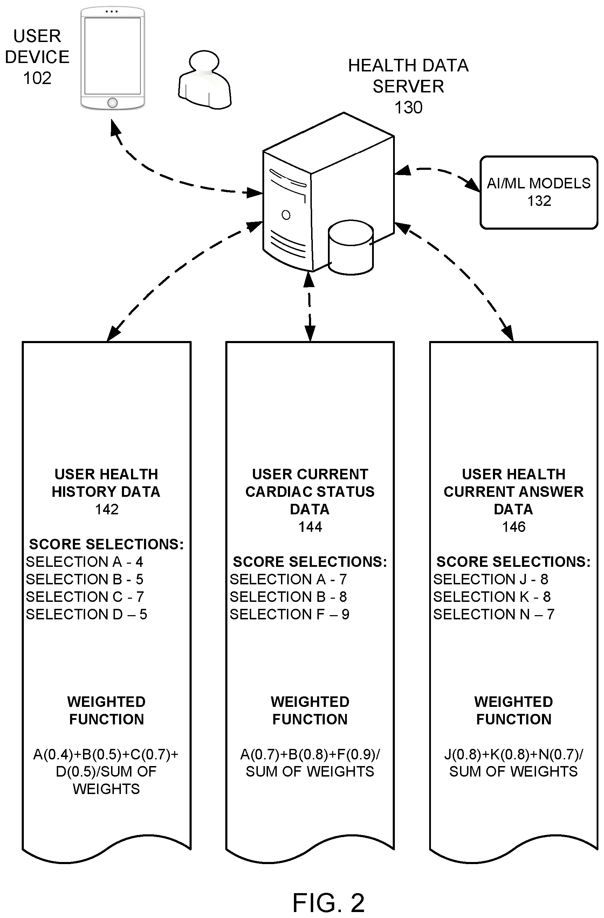

FIG. 2 illustrates an example of a data collecting and processing model used for creating a data report and determining whether a user is having medical problems according to example embodiments. Referring to FIG. 2, the example illustrates how data from three primary sources can be used to identify a user's current health condition. The health data server 130 may be a source of user health data including current, past and automatically derived data, such as the facial scan data. The AI and/or machine learning (ML) data models 132 may include one or more software routines which populate with the user health data and provide dynamic results indicating a user's current health status. The three main categories of health data may be considered 142, which is based on historical health data including previously stored data points, current cardiac data based on the facial scan analysis 144 and current health related answers 146 to questions prompted to the user via the application. The results generated from the various data sources may be a function of all three. Each data set may be based on numerical status data, such as 1-10 in terms of severity and/or the assigned tags including all four levels of urgency. The weighted functions of each data source may be combined into a single health function, such as health score (HS) = history data (HD) + cardiac data (CD) + current answer data (CAD). Each of the three data segments may be based on a weighted function of each of the data parameters included in the data set. As the value of the weights increase, the overall health score may increase in a proportional manner.

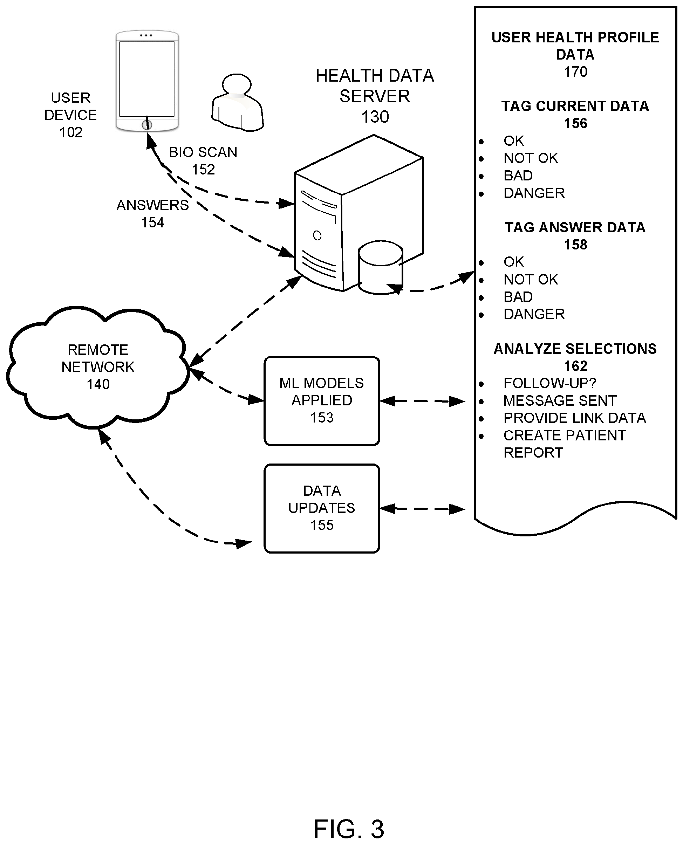

FIG. 3 illustrates an example of a data collecting and processing procedure used for collecting data and outputting a medical report based on a machine learning model(s) according to example embodiments. Referring to FIG. 3, the process begins with a user accessing a user device 102 to initiate a bio-scan 152 of the user's face. At some point, the application may ask questions, such as when the facial data is being processed. The process of obtaining more health information may offers updates and increased accuracy when summarizing the user's present health status. The data stored may be part of a profile 170 that includes tagged data representing the current data 156, such as the cardiovascular scan data, the other data portions may include tagged data 158, such as the question and answer data and the results derived from the health profile may include selections to notify 162 the user and/or other interested parties, such as significant others, hospitals, physician offices, etc. The data in the profile 170 may be subjected to ML models 152 which are trained and updated to make decisions regarding whether the user needs certain care or other services. The models 152 may generate new data and may normalize existing data for interpretation purposes and provide the data in update messages 154. A remote network 140 in the cloud may provide data services to the user application for processing and data storage as well as applying ML models and other data structures to the user profile data.

The application may evaluate whether a message, communication or other type of alert should be issued to the patient, the physician, an interested party or all of the above. If action is warranted based on a user's current health score, the platform constructs a multi-part outbound message that may include an action message with a hyperlink to a physician service center (e.g., request to schedule a visit), a comment or informational message to contextualize the status (e.g., you are at risk of heart attack or stroke, etc.), and/or a footer message containing additional reference or educational material. The data are collectively sent via a secure delivery mechanism to patients and providers and/or others. In the case of clinician notifications, the application also prepares a structured summary of the logic that triggered the alert, along with contributing biometric and self-reported factors for evidentiary purposes.

The instant solution may construct an outbound message using a structured message template stored in the memory. The engagement and interaction manager 230 may retrieve the structured message template and may populate the structured message template with the cardiovascular score, the consolidated tag set, and the inference output to produce a structured message. The structured message may include a header field identifying a recipient endpoint, a body field including health condition data, and an action field specifying a recommended action.

The instant solution may transmit the structured message to a recipient endpoint using a communication interface. The engagement and interaction manager 230 may retrieve a routing rule from the memory and may select the recipient endpoint based on the consolidated tag set and the inference output. The communication interface may transmit the structured message to the recipient endpoint, wherein the recipient endpoint includes at least one of a clinician server, a monitoring dashboard, or a mobile notification service.

Example of health related metrics may include blood pressure (systolic and diastolic), heart rate, HRV and cardiac stress, breathing rate, cardiac workload, parasympathetic activity, BMI, blood glucose, HbA1c, and additional composite health indices as needed. The application is designed to enable continuous monitoring and scalable intervention, adapting dynamically to evolving patient states over time, by leveraging historical trends and real-time updates to drive personalized, timely clinical engagement.

One example may include the process beginning when a user-facing camera captures real-time facial video/images. The camera may be integrated into a smartphone, tablet, laptop, smart mirror, and/or a dedicated health monitoring kiosk. The video capture function initiates recording upon detecting a human face within frame boundaries, confirmed by a facial landmark recognition algorithm. Environmental readiness is validated through a luminance thresholding check that ensures adequate lighting. No physical interaction is required from the user beyond remaining stationary and visible to the camera. Following the data metric extraction procedure, each parameter is classified into one of four color-coded health categories: green (normal), yellow (borderline), orange (abnormal), and red (critical). Classification thresholds are calculated by referencing individualized baselines informed by user profile data (e.g., age, sex, known diagnoses) and further refined by historical scan trends which may be supported by ML models. A rule-based inference engine evaluates combinations of tags to identify composite risk patterns and initiate downstream actions.

The AI model that supports the application functionalities is trained on a hierarchically structured dataset. A first tier comprises high-resolution annotated facial recordings linked to gold-standard biometric measurements from clinical-grade instruments. The second tier contains low-to-medium resolution video records spanning a diverse user base, annotated using a probabilistic ensemble of bio-signal estimators. The model training process integrates supervised learning (for metric prediction), semi-supervised anomaly detection, and unsupervised clustering for identifying novel signal patterns.

The instant solution may construct a feature tensor for input to the AI health analytics engine 220, wherein the feature tensor comprises a temporal sequence of normalized metric values, tag indicators, and historical trend values. The processing unit 604 may retrieve the feature tensor from the memory and may provide the feature tensor to the AI health analytics engine 220 for inference.

The instant solution may produce an inference output comprising a predicted health status score and a confidence value. The AI health analytics engine 220 may store the inference output in the memory and may transmit the inference output to the engagement and interaction manager 230, wherein the inference output may be used to determine an action state associated with a health condition.

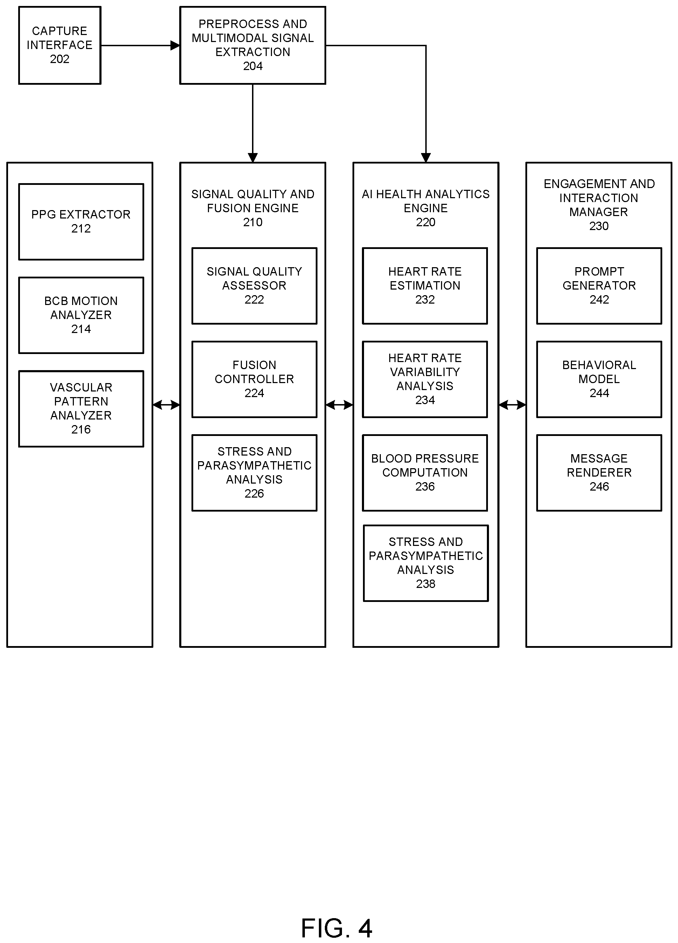

FIG. 4 illustrates an example logic diagram illustrating information and data processing modules used to measure and monitor according to example embodiments. Referring to FIG. 4, the example provides an initial image capture interface function 202 which permits a user to submit video and/or photos of their face or other body parts to be scanned and analyzed for health data metrics. The preprocess and multimodal functions perform signal extraction 204 to a signal quality and fusion engine 210 and for an AI health analytics engine 220.

The architecture comprises distinct functional blocks interconnected by directional data flows that are explicitly labeled to indicate the content and context of each transmission. The application begins with a capture interface that acquires a facial video stream from a user-facing camera embedded in a smartphone, tablet, laptop, or kiosk device. This video stream may include temporally aligned facial image frames, which are transmitted to the preprocessing and multimodal signal extraction module 204, which performs image normalization, facial landmark detection, and segmentation of the facial region across frames, and outputs frame-based facial signal segments, which are distributed in parallel to multiple bio-signal extraction pathways.

The instant solution may select a subset of frames from the facial image frames received by the preprocessing and multimodal signal extraction module 204 using a frame selection rule stored in the memory. The preprocessing and multimodal signal extraction module 204 may determine a stability metric for the facial image frames based on inter-frame motion magnitude, facial landmark displacement, and brightness variation, and may select the subset of frames that satisfy the stability metric. The subset of frames may be stored in the memory with associated timestamp indices and may be provided to downstream bio-signal extraction pathways.

The instant solution may align the subset of frames across processing pathways using a synchronization index derived from the timestamp indices. The preprocessing and multimodal signal extraction module 204 may assign the synchronization index to the subset of frames and may generate aligned frame groups, wherein the aligned frame groups include temporally corresponding frames for parallel processing by the PPG extractor 112, the BCG motion analyzer 214, and the vascular pattern analyzer 216. The aligned frame groups may be transmitted to the signal quality and fusion engine 210 for consistent temporal analysis.

The extracted segments are received by specialized modules including a PPG extractor 112 that analyzes temporal pixel intensity variations to derive photoplethysmographic waveforms, a BCG motion analyzer 214 that captures micro-movements of the facial surface resulting from cardiac mechanical ejection using motion vector decomposition, and a vascular pattern analyzer 216 that evaluates changes in facial texture and contrast to estimate blood flow direction and velocity. Each of these modules outputs raw bio-signal data streams, comprising time series that reflect physiological pulsations or flow dynamics.

The raw streams are transmitted to the signal quality and fusion engine 210 where a signal quality assessor 222 computes modality-specific confidence scores based on a signal-to-noise ratio(s), facial stability, lighting consistency, and occlusion detection. A fusion controller 224 receives the quality metrics and selects either a dominant signal or a weighted combination, depending on environmental and user-specific conditions. A stress and parasympathetic analysis function 226 identifies stress indicators. The output of is a set of fused bio-signal metrics and a clean and integrated physiological signal representation.

The instant solution may determine a modality selection using a feature vector derived from signal quality metrics. The signal quality assessor 222 may generate a feature vector comprising a signal-to-noise ratio value, a motion stability value, a luminance consistency value, and an occlusion probability value. The fusion controller 224 may retrieve a decision model from the memory and may apply the decision model to the feature vector to produce a modality selection output.

The instant solution may assign a weight distribution to the raw bio-signal data based on the modality selection output. The fusion controller 224 may generate a weight vector corresponding to the raw bio-signal data and may apply the weight vector to produce a fused biosignal output. The fused bio-signal output may be transmitted to the AI health analytics engine 220 as a unified physiological signal representation.

The fused data metrics are passed to the AI health analytics engine 220, where they undergo advanced biometric interpretation. The engine 220 comprises four core analytic modules including heart rate estimation 232, which identifies peaks in the time series to calculate beats per minute, heart rate variability analysis 234, which derives time-domain and frequency-domain HRV indices, blood pressure computation 236, which estimates systolic and diastolic pressure using pulse transit time and regression-based personalization, and stress and parasympathetic analysis 238, which isolates high-frequency HRV components and applies spectral analysis to infer autonomic nervous system activity. These outputs are annotated with risk categories, and the engine generates a labeled set of health indices and tags for interpretation and feedback.

The health indices and tags are forwarded to the engagement and interaction manager 230. This module governs the delivery of insights to users and the generation of responsive prompts. It includes a prompt generator 242 that issues questions or reminders based on biometric triggers, a behavioral model 244 that tracks engagement metrics, such as scan frequency and/or trend stability, and a message renderer 246 that creates feedback messages personalized to the user's context and condition(s).

In another example, when the AI module(s) detect metric anomalies, such as an elevated stress index combined with abnormal cardiac workload, the prompt generator 242 may implement adaptive user-facing prompts, such as "Are you experiencing pain or emotional distress?" User responses are parsed in real time by the behavioral model 244, which acts as a contextual feedback engine. This model dynamically adjusts the inference weights within the AI health analytics engine 220 and updates the associated health tags through the stress and parasympathetic analysis module 226, enabling refined risk categorization tailored to each user's condition.

In another example, the application evaluates escalation criteria using statistical thresholds derived from the heart rate variability analysis 234 and blood pressure computation 236 modules. If systolic pressure remains elevated across multiple rolling windows while parasympathetic signals remain suppressed, the engagement and interaction manager 230 initiates a triage recommendation. This escalation logic is governed by a Bayesian risk model that integrates real-time outputs and longitudinal volatility scores to generate action confidence scores. Depending on severity, the analysis may notify the user, trigger a clinician alert via a secure API endpoint, or log the event to an anomaly record within the user's health timeline.

Monitoring is enabled through the behavioral model 244 and underlying time-series storage. Metric trends are segmented into rolling windows, such as 10-day, 30-day, and 90-day and analyzed for trends, such as slope, volatility, and inflection points. The data analytics feed into an adaptive threshold that calibrates alerting logic to the user's baseline physiology. For instance, a user with persistently low HRV may have elevated alert thresholds to prevent false positives, while abrupt deviations still activate downstream triage. Risk strategy is enhanced by incorporating non-biometric data sources into an inference pipeline. The AI health analytics engine 220 receives supplemental variables, such as smoking history, lipid profile, diabetes status, and prescribed medications, which may be entered via the user interface or imported through the FHIR-compatible API endpoint (not shown). These additional variables are integrated into validated risk scoring frameworks (e.g., Framingham, SCORE) and cross-referenced against live biometric data to generate a composite wellness score.

The data results are delivered through an interactive user interface powered by the message renderer 246. The interface presents multivariate plots, historical compliance badges, and dynamic messages. In one example, a user may see, "Your average BP has improved this week, continue your current plan." In clinical mode, structured alerts include explanatory tags, such as, "Red systolic pressure confirmed across 3 scans, combined with increased RPP and HRV suppression, suggesting acute cardiac strain," with traceability back to source metrics. The condition tags and escalation decisions are exportable via secure, FHIR-compatible API endpoints (not shown). Output formats include FHIR, observations, HL7 bundles, and encrypted JSON packets. Transmission is secured through OAuth2.0 scopes and logged with audit controls. If full EHR integration is unavailable, a smart-on-FHIR session link provides secure, time-limited access for clinicians without backend storage.

In one example, the application may assign behavioral tags to user responses and biometric readings to support dynamic triage logic. For instance, if a user submits localized pain and their stress index exceeds a predefined threshold, the behavioral model 244 classifies the event as potentially acute. The application then uses the message renderer 246 to compile a structured summary and flags the record for clinician review. In the automated version, the engagement and interaction manager 230 bypasses user confirmation and directly initiates a scheduling request through a provider's API based on urgency and calendar availability.

In another example, the application detects that an elevated biometric indicator may originate from a domain outside the monitoring areas. While tracking hypertension, for example, the AI health analytics engine 220 may identify a signature indicative of gastrointestinal stress and elevated cardiac workload without corresponding BP deviation, and trigger diagnostic branching via the prompt generator 242. A new set of diagnostic questions may surface automatically, and the triage algorithm is updated without clinician input. To reinforce user engagement, the behavioral model 244 tracks scan frequency and quality (e.g., 70% scan compliance over 30 days). When engagement activity exceeds preset thresholds (e.g., 65%), the message renderer 246 delivers positive reinforcement, such as "Great job! You've completed a 7-day scan streak." The application then predicts future risk using regression models and time-series forecasting. Notifications may be issued proactively using insights from user-specific engagement patterns.

In another embodiment, the application identifies complex multi-specialty patterns, for example, concurrent elevation in vascular age, low HRV, and high BMI. The application simultaneously routes the case to cardiology and endocrinology using parallel message queues, each formatted with domain-relevant metrics. The routing logic is managed by the fusion controller 224 and engagement layer.

Tagging thresholds (e.g., normal/OK, borderline/not-OK, abnormal/Bad, critical/Danger) may evolve through machine-learned personalization. Over a 90-day window, persistent borderline HRV with improving BP may prompt the AI health analytics engine 220 to recalibrate the HRV threshold upward, reducing alert noise. Retraining may be optionally executed on-device to improve inference accuracy without external data transmission. In passive facility use cases (e.g., eldercare), the platform may operate in a background mode of operation. The capture interface 202 activates automatically during natural routines (e.g., meals), and facial scans are silently analyzed. If anomalies are detected, the message renderer 246 logs alerts to a local caregiver dashboard.

In one example, a sudden increase in respiratory irregularities may indicate a viral outbreak. The AI health analytics engine 220 may flag the event and generate a report for public health agencies. Anomaly clustering is continuously performed using dispersion and density thresholds, eliminating the need for explicit population-level labeling. In another example, the platform is deployed as a zero-install, browser-based interface. Users may launch the session by scanning a QR code or clicking a link, triggering the capture interface 202 and preprocessing module 204 in-browser. Multimodal signal extraction, including fusion and classification, may be performed locally via WebAssembly (WASM) and/or JavaScript. In an autonomous variant, the engagement and interaction manager 230 generates session links based on user history and distributes them automatically via secure messaging.

For signal robustness, the application incorporates a modality substitution framework. The signal quality assessor 222 monitors confidence scores across PPG, BCG, and vascular mapping in real time. When one modality degrades (e.g., due to low lighting), the fusion controller 224 reroutes analytic priority and recomputes weighted contributions. This fallback logic adapts based on facial geometry, ambient conditions, and past scan behavior. To further ensure data fidelity, the platform overlays a real-time facial grid via the preprocessing module 204. Each data metric is scored for reliability, and unstable data is dynamically masked from analysis. In the automated variant, the grid resolution and weighting are adapted mid-scan to optimize usable signal coverage. Users with metabolic syndrome receive panels prioritizing BMI, and stress, while hypertensive users see vascular and BP metrics. The message renderer 246 selects from dynamic templates and composes the final report view. When exporting to clinical systems, the platform detects available EHR capabilities via metadata or redirect headers. The export layer then selects between a full FHIR bundle, a launch link, and/or a structured summary to ensure compatibility without backend customization.

Real-time scan quality monitoring is performed by an instruction generation module within the prompt generator 242. If the user is misaligned, exposed to poor lighting, or moving excessively, the system issues corrective prompts with visual cues. In the predictive variant, timing and specificity are tuned based on historical user correction rates. In another example, the platform includes a trigger template library. Each template defines compound biometric conditions that map to escalation paths. For example, a health condition template requiring SBP > 140, HRV < 30 ms, and age > 65 may trigger a tier-2 alert routed through the message renderer 246. Templates are continuously adapted.

In a further example, the instant solution may include a confidence-weighted multi-pass inference engine that dynamically reprocesses biometric data when initial metrics fall within a borderline or ambiguous range. After the first analytic pass using the biosignal metrics, when one or more outputs are tagged as borderline with low confidence, the application autonomously reexecutes the fusion and analysis pipeline with adjusted signal weights and extended temporal segments. This recursive evaluation leverages contextual metadata, such as ambient lighting shifts, motion patterns, and historical variance profiles, to reweigh modality contributions (e.g., favoring BCG over PPG in low light). This functionality increases diagnostic resolution and reduces inconclusive outcomes, especially in edge cases where data quality fluctuates during the scan.

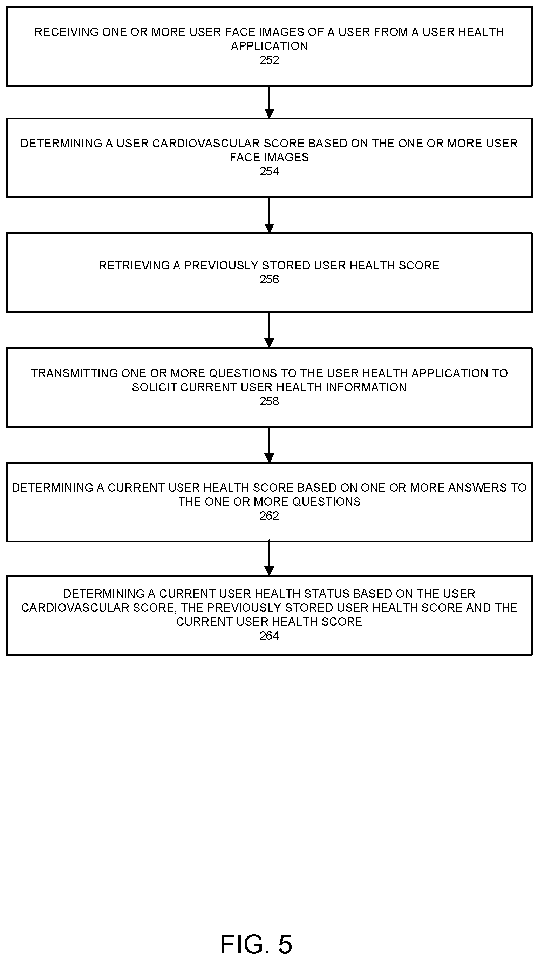

FIG. 5 illustrates an example flow diagram of an example process of measuring and monitoring patient health according to example embodiments. Referring to FIG. 5, the example provides receiving one or more user face images and/or video from a user device application operating a health application 252. The process may also include determining a user cardiovascular score 254 based on the one or more user face images based on comparisons between the images. The information being analyzed may rely on digital imaging pixelations to identify heart related data metrics. Once the cardiovascular data is identified, the process may include retrieving a previously stored user health score 256 based on previously stored cardiovascular data, user inputted answer data, user records associated with previous doctor visits, etc. The application may attempt to identify more current data by submitting questions and prompting the user via the user interface to answer the questions. The new data received may be scored, tagged and stored in memory as a portion of the final health status update determination. The process may also include transmitting one or more questions to the user health application to solicit current user health information 258, determining a current user health score based on one or more answers to the one or more questions 262 and determining a current user health status based on the user cardiovascular score, the previously stored user health score and the current user health score 264.

The health status may be something polarized, such as no action vs. seek medical attention or immediate medical attention. The process may include using the current cardiovascular information as a weighted decision parameter, such as one third of the total weight, and the question and answer data as one third the total weight and history information as one third the total weight as part of the current user health status determination. The three scores may be generated and summed together and then compared to a threshold value to identify if imminent and immediate health care services are needed.

The process may also include performing a photoplethysmography and ballistocardiography analysis on the more or more user face images to identify a plurality of cardiovascular metrics. Those processing operations may include digital processing of the image data and comparing the various frames of image data to one another to measure and estimate various cardiovascular metrics.

The plurality of cardiovascular metrics may be combined into a scoring function which is used to calculate a risk score. The plurality of cardiovascular metrics may include heart rate, heart rate variability, stress index, and blood pressure, and other metrics may be used as well. The process may also include assigning a tag indicating a health severity level to each of the plurality of cardiovascular metrics, and assigning a tag to each of a plurality of health metrics extracted from the one or more answers. The tags may be one or four categories of urgency related to the user's health.

The instant solution may assign a normalized metric value to the plurality of cardiovascular metrics by mapping raw signal outputs to a bounded scale using a calibration table stored in a memory. The calibration table may associate a physiological measurement range with a normalized score value, wherein the normalized score value corresponds to a defined clinical range. The AI health analytics engine 220 may retrieve the calibration table from the memory and may apply a transformation to convert the physiological measurement into the normalized metric value.

The instant solution may determine a cardiovascular score by combining the normalized metric value using a weighted aggregation structure. The AI health analytics engine 220 may retrieve a weight vector from the memory, wherein the weight vector includes a weight assigned to the normalized metric value. The AI health analytics engine 220 may multiply the normalized metric value by the weight to produce a weighted metric value and may sum the weighted metric value to produce the cardiovascular score. The cardiovascular score may be stored in the memory and may be transmitted to the engagement and interaction manager 230 for downstream processing.

The instant solution may assign a tag to the normalized metric value using a threshold comparison structure. The AI health analytics engine 220 may retrieve a threshold set from the memory, wherein the threshold set includes a lower bound and an upper bound associated with a tag category. The AI health analytics engine 220 may compare the normalized metric value to the threshold set and may assign a tag label corresponding to the threshold range.

The instant solution may resolve conflicting tag assignments across the plurality of cardiovascular metrics by applying a priority rule stored in the memory. The priority rule may define a hierarchy among tag categories and may select a dominant tag based on the hierarchy. The AI health analytics engine 220 may apply the priority rule to produce a consolidated tag set, wherein the consolidated tag set may be transmitted to the engagement and interaction manager 230 for action determination.

The process may also include determining whether any of the tags indicate immediate health care service, and wherein when none of the tags indicate immediate health care service, the process may also include determining whether the current user health status has exceeded a health score threshold and when the current user health status has exceeded the health score threshold, then creating a message indicating a need for immediate health care.

The content of a ML model may include all information in the default question template and corresponding answers, a user profile of known health information about the user, profile information of other users from previous communication sessions, available information sources, such as health and medical databases, government demographic information, third party websites, etc. As information is received from a user during a biometric scan and/or questionnaire format, the ML model may begin comparing the information retrieved to information in the ML model. The ML model data may then be increased, decreased or modified depending on the objective criteria used to define the ML model. The ML model may also have a set of rules and objectives, such as semantic words and phrases, mathematical expressions, etc., which define how the model is used and what rules are to be employed during data analysis operations. The ML model may be the basis for limiting data results and performing other operations necessary to obtain the query information used by the application. The ML model is used as the basis to create a set of questions, change the set of questions, apply weights to the questions, create new questions and to limit a list of questions for the default template and create modified question templates based on user feedback.

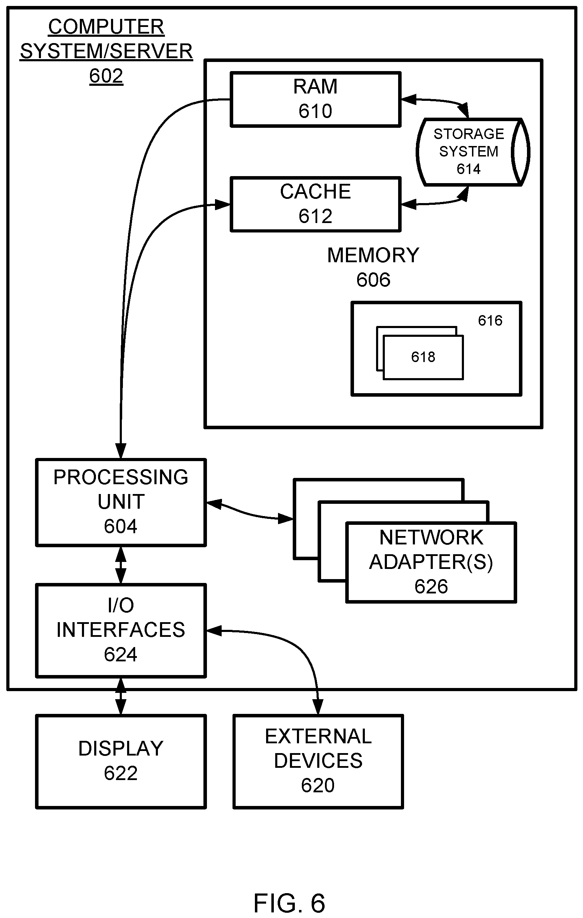

FIG. 6 illustrates an example network entity device configured to store instructions, software, and corresponding hardware for executing the same according to example embodiments. FIG. 6 is not intended to suggest any limitation as to the scope of use or functionality of embodiments of the application described herein. Regardless, the computing node 600 is capable of being implemented and/or performing any of the functionality set forth hereinabove.

In computing node 600 there is a computer system/server 602, which is operational with numerous other general purpose or special purpose computing system environments or configurations. Examples of well-known computing systems, environments, and/or configurations that may be suitable for use with computer system/server 602 include, but are not limited to, personal computer systems, server computer systems, thin clients, rich clients, hand-held or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server 602 may be described in the general context of computer systemexecutable instructions, such as program modules, being executed by a computer system. Generally, program modules may include routines, programs, objects, components, logic, data structures, and so on that perform particular tasks or implement particular abstract data types. Computer system/server 602 may be practiced in distributed cloud computing environments where tasks are performed by remote processing devices that are linked through a communications network. In a distributed cloud computing environment, program modules may be located in both local and remote computer system storage media including memory storage devices.

As displayed in FIG. 6, computer system/server 602 in cloud computing node 600 is displayed in the form of a general-purpose computing device. The components of computer system/server 602 may include, but are not limited to, one or more processors or processing units 604, a system memory 606, and a bus that couples various system components including system memory 606 to processor 604.

The bus represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnects (PCI) bus.

Computer system/server 602 typically includes a variety of computer system readable media. Such media may be any available media that is accessible by computer system/server 602, and it includes both volatile and non-volatile media, removable and non-removable media. System memory 606, in one embodiment, implements the flow diagrams of the other figures. The system memory 606 can include computer system readable media in the form of volatile memory, such as random-access memory (RAM) 610 and/or cache memory 612. Computer system/server 602 may further include other removable/non-removable, volatile/non-volatile computer system storage media. By way of example only, storage system 614 can be provided for reading from and writing to a non-removable, non-volatile magnetic media (not displayed and typically called a "hard drive"). Although not displayed, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to the bus by one or more data media interfaces. As will be further depicted and described below, memory 606 may include at least one program product having a set (e.g., at least one) of program modules that are configured to carry out the functions of various embodiments of the application.

Program/utility 616, having a set (at least one) of program modules 618, may be stored in memory 606 by way of example, and not limitation, as well as an operating system, one or more application programs, other program modules, and program data. Each of the operating system, one or more application programs, other program modules, and program data or some combination thereof, may include an implementation of a networking environment. Program modules 618 generally carry out the functions and/or methodologies of various embodiments of the application as described herein.

As will be appreciated by one skilled in the art, aspects of the present application may be embodied as a system, method, or computer program product. Accordingly, aspects of the present application may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present application may take the form of a computer program product embodied in one or more computer readable medium(s) having computer readable program code embodied thereon.

Computer system/server 602 may also communicate with one or more external devices 620 such as a keyboard, a pointing device, a display 622, etc.; one or more devices that enable a user to interact with computer system/server 602; and/or any devices (e.g., network card, modem, etc.) that enable computer system/server 602 to communicate with one or more other computing devices. Such communication can occur via I/O interfaces 624. Still yet, computer system/server 602 can communicate with one or more networks such as a local area network (LAN), a general wide area network (WAN), and/or a public network (e.g., the Internet) via network adapter(s) 626. As depicted, network adapter(s) 626 communicates with the other components of computer system/server 602 via a bus. It should be understood that although not displayed, other hardware and/or software components could be used in conjunction with computer system/server 602. Examples include, but are not limited to: microcode, device drivers, redundant processing units, external disk drive arrays, RAID systems, tape drives, and data archival storage systems, etc.

The instant solution may partition processing operations between the user device 102 and the health data server 130 using a task allocation structure stored in the memory. The user device 102 may execute the preprocessing and multimodal signal extraction module 204 and may transmit a processed signal packet to the health data server 130 via the network adapter 626. The processed signal packet may include the normalized metric value and associated signal quality metrics.

The health data server 130 may receive the processed signal packet and may execute the AI health analytics engine 220 to produce the inference output. The health data server 130 may transmit a result packet to the user device 102, wherein the result packet may include the cardiovascular score, the consolidated tag set, and the inference output. The user device 102 may receive the result packet and may provide the result packet to the engagement and interaction manager 230 for message rendering.

One skilled in the art will appreciate that a "system" could be embodied as a personal computer, a server, a console, a personal digital assistant (PDA), a cell phone, a tablet computing device, a smartphone or any other suitable computing device, or combination of devices. Presenting the above-described functions as being performed by a "system" is not intended to limit the scope of the present application in any way but is intended to provide one example of many embodiments. Indeed, methods, systems and apparatuses disclosed herein may be implemented in localized and distributed forms consistent with computing technology.

It should be noted that some of the system features described in this specification have been presented as modules, in order to more particularly emphasize their implementation independence. For example, a module may be implemented as a hardware circuit comprising custom very largescale integration (VLSI) circuits or gate arrays, off-the-shelf semiconductors such as logic chips, transistors, or other discrete components. A module may also be implemented in programmable hardware devices such as field programmable gate arrays, programmable array logic, programmable logic devices, graphics processing units, or the like.

A module may also be at least partially implemented in software for execution by various types of processors. An identified unit of executable code may, for instance, comprise one or more physical or logical blocks of computer instructions that may, for instance, be organized as an object, procedure, or function. Nevertheless, the executables of an identified module need not be physically located together but may comprise disparate instructions stored in different locations which, when joined logically together, comprise the module and achieve the stated purpose for the module. Further, modules may be stored on a computer-readable medium, which may be, for instance, a hard disk drive, flash device, random access memory (RAM), tape, or any other such medium used to store data.

Indeed, a module of executable code could be a single instruction, or many instructions, and may even be distributed over several different code segments, among different programs, and across several memory devices. Similarly, operational data may be identified and illustrated herein within modules and may be embodied in any suitable form and organized within any suitable type of data structure. The operational data may be collected as a single data set or may be distributed over different locations including over different storage devices, and may exist, at least partially, merely as electronic signals on a system or network.

It will be readily understood that the components of the application, as generally described and illustrated in the figures herein, may be arranged and designed in a wide variety of different configurations. Thus, the detailed description of the embodiments is not intended to limit the scope of the application as claimed but is merely representative of selected embodiments of the application.

One having ordinary skill in the art will readily understand that the above may be practiced with steps in a different order, and/or with hardware elements in configurations that are different than those which are disclosed. Therefore, although the application has been described based upon these preferred embodiments, it would be apparent to those of skill in the art that certain modifications, variations, and alternative constructions would be apparent.

While preferred embodiments of the present application have been described, it is to be understood that the embodiments described are illustrative only and the scope of the application is to be defined solely by the appended claims when considered with a full range of equivalents and modifications (e.g., protocols, hardware devices, software platforms etc.) thereto.

In this specification, reference is made in detail to specific embodiments of the invention. Some of the embodiments or their aspects are illustrated in the drawings.

For clarity in explanation, the invention has been described with reference to specific embodiments, however it should be understood that the invention is not limited to the described embodiments. On the contrary, the invention covers alternatives, modifications, and equivalents as may be included within its scope as defined by any patent claims. The following embodiments of the invention are set forth without any loss of generality to, and without imposing limitations on, the claimed invention. In the following description, specific details are set forth in order to provide a thorough understanding of the present invention. The present invention may be practiced without some or all of these specific details. In addition, well known features may not have been described in detail to avoid unnecessarily obscuring the invention.

In the foregoing disclosure, implementations of the disclosure have been described with reference to specific example implementations thereof. It will be evident that various modifications may be made thereto without departing from the broader spirit and scope of implementations of the disclosure as set forth in the following claims. The disclosure and drawings are, accordingly, to be regarded in an illustrative sense rather than a restrictive sense.

## Claims

1. A method comprising:
receiving one or more user face images of a user from a user health application;
determining a user cardiovascular score based on the one or more user face images;
retrieving a previously stored user health score;
transmitting one or more questions to the user health application to solicit current user health information;
determining a current user health score based on one or more answers to the one or more questions; and
determining a current user health status based on the user cardiovascular score, the previously stored user health score and the current user health score.

2. The method of claim 1, further comprising
performing a photoplethysmography and ballistocardiography analysis on the more or more user face images to identify a plurality of cardiovascular metrics.

3. The method of claim 2, wherein the plurality of cardiovascular metrics are combined into a scoring function which is used to calculate a risk score.

4. The method of claim 2 or 3, wherein the plurality of cardiovascular metrics include heart rate, heart rate variability, stress index, and blood pressure.

5. The method of any of claims claim 2 to 4, comprising
assigning a tag indicating a health severity level to each of the plurality of cardiovascular metrics;
assigning a tag to each of a plurality of health metrics extracted from the one or more answers.

6. The method of claim 5, comprising
determining whether any of the tags indicate immediate health care service.

7. The method of claim 5 or claim 6, wherein when none of the tags indicate immediate health care service, determining whether the current user health status has exceeded a health score threshold.

8. The method of claim 7, wherein when the current user health status has exceeded the health score threshold, then creating a message indicating a need for immediate health care.

9. A computer-readable storage medium comprising instructions which, when executed by one or more processors of a computer system, cause the computer system to perform:
receiving one or more user face images of a user from a user health application;
determining a user cardiovascular score based on the one or more user face images;
retrieving a previously stored user health score;
transmitting one or more questions to the user health application to solicit current user health information;
determining a current user health score based on one or more answers to the one or more questions; and
determining a current user health status based on the user cardiovascular score, the previously stored user health score and the current user health score.

10. The computer-readable storage medium of claim 9, wherein the instructions, when executed by the one or more processors of the computer system, cause the computer system to perform the method of any of claims 2 to 8.

11. A computer system comprising one or more processors configured to:
receive one or more user face images of a user from a user health application;
determine a user cardiovascular score based on the one or more user face images;
retrieve a previously stored user health score;
transmit one or more questions to the user health application to solicit current user health information;
determine a current user health score based on one or more answers to the one or more questions; and
determine a current user health status based on the user cardiovascular score, the previously stored user health score and the current user health score.

12. The computer system of claim 11, wherein the one or more processors is configured to perform the method of any of claims 2 to 8.

13. The computer system of claim 11 or claim 12, wherein the one or more processors comprises one or more server.

14. The computer system of any of claims 11 to 13, wherein the one or more processors comprises a user device running the user health application.

15. The computer system of any of claims 11 to 14, further comprising the computer readable storage medium of claim 9 or claim 10.
